# EUROPEAN PATENT SPECIFICATION

(11) **EP 2 926 639 B1**
(45) Date of publication and mention of the grant of the patent: **09.11.2016**
(21) Application number: 14196786.9
(22) Date of filing: 08.12.2014
(51) Int. Cl.: A01C 5/02, A01G 9/08, A01H 4/00

(54) **METHOD AND DEVICE FOR PRICKING OUT PLANTS**
VERFAHREN UND VORRICHTUNG ZUM AUSSETZEN VON PFLANZEN
PROCÉDÉ ET DISPOSITIF DE REPIQUAGE DE VÉGÉTAUX

(30) Priority: 02.04.2014 CZ 20140215
(43) Date of publication of application: 07.10.2015
(73) Proprietor: Mendelova Univerzita v Brne, 61300 Brno (CZ)
(72) Inventor: Stritecky, Josef, 67551 Jaromerice nad Rokytnou (CZ); Cafourek, Josef, 67552 Lipnik (CZ); Madera, Petr, 61300 Brno (CZ)
(74) Representative: Hartvichova, Katerina

(56) References cited:
- WO-A1-2015/097603
- US-A- 982 575
- US-A- 1 563 625
- US-A- 4 031 832

## Description

### Field of Art

The present invention refers to a method of pricking out (transplanting) plants in which the root system of a pricked-out plant is placed into the growing substrate (e.g., potting soil).

### Background Art

Current practice in pricking out seedlings involves inserting the root system of a plant (i.e. the underground part of the plant) into a pre-prepared cavity or hollow space in the growing substrate (e.g., potting soil). Then the root system of the plant (i.e. the underground part of the plant) is pressed in the growing substrate; this step closes up the cavity and fuses the root system of the plant with the surrounding growing substrate.

Very often this practice leads to undesirable permanent deformation of the plant's roots.

It is caused by the fact that during the placing of the root system into the growing substrate as described above, the supple root tip bends up into the opposite direction and forms a pipe-shaped deformation. This is not a fatal problem for vegetables and flowers, however, forest trees and shrubs form root aggregates after several years, and these root aggregates cause damages to a high percentage of forest trees and shrubs. Such damaged plants decline, slow down their development or they might be uprooted, windthrowned, and wither up.

Another way of pricking out seedlings is known as the so-called horizontal pricking out, in between two layers of the growing substrate. This method is very reliable, however, it requires the use of special cultivating caskets, for example caskets "Patrik", resulting in an increase of costs of cultivation of the seedlings.

Furthermore, a mechanized method of transplanting the seedlings is known in the art, said method being substantially a combination of manual insertion of the seedlings into a carrier, followed by partial rotation or another mechanical movement of the carrier, which places the root systems of the seedlings into pre-prepared cavities in the growing substrate. The process of the uniform planting of the cultivation furrow is terminated by closing when these pre-prepared cavities are closed by pressure applied by pressing elements. Even this method does not fully eliminate deformations of the root system because the plant's root system is inserted into the growing substrate in a direction opposite to the growth direction. The more the primary root of the seedling is soft and malleable, the more considerable the root deformation can be.

Devices for and methods of pricking out plants are shown in US 982 575 A, US 1 563 625 A, US 4 031 832 A and WO 2015/097603 A1.

The aim of the present invention is to overcome the drawbacks of the prior art.

### Disclosure of the Invention

The present invention overcomes the above described problems by providing a novel method of pricking out plants, wherein the plant's root system is inserted into guiding means of a pricking-out device. The root system is inserted in a straightened position, i.e., the roots are not bent or tilted. The pricking-out device together with the plant's root system are subsequently passed into a layer of growing substrate by a unidirectional movement. In a desired depth within the layer of the growing substrate, the plant's root system is released from the guiding means, and the pricking-out device continues to move in the direction of the unidirectional movement out of the layer of the growing substrate.
The pricking-out device thus passes through the whole layer of the growing substrate, while the plant's root system is released in the desired depth within the layer of the growing substrate. As the pricking-out device with the guiding means passes through the whole layer of the growing substrate by a unidirectional movement, it guides the root system of the plant into the substrate in a straightened position and does not disturb this position after the release of the root system from the guiding means, thereby preventing bending or tilting of the roots.

In one preferred embodiment, the guiding means of the device for the method according to the present invention contain a substantially flexible longitudinal flat body, which enables to enwrap, surround or enclose the root system of the pricked out plant prior to its introduction into the growing substrate.

In another preferred embodiment, the prick of the device for the method according to the present invention is needle-shaped.

The present invention excludes a counter sense motion of the root or the root system of the pricked out plant (i.e., a motion of the roots, parts of the root system or the whole root system in a direction opposite to the direction of insertion of the root system into the growing substrate) during the pricking out process. This fact has a positive effect on the quality of the pricked-out seedlings and tree nursery seedlings, as well as on their growth in further cultivations.

The present invention in particular increases the production efficacy of the woody plant seedlings with low seed germination capacity. It is therefore possible to divide the production of the enwrapped seedlings into a seed germination step and into a step involving pricking out seedlings into containers using the method and device according to the present invention, which renders the tree nursery technology, irrigation, protective and fertilizing spraying more cost-effective.

### Brief Description of the Drawings

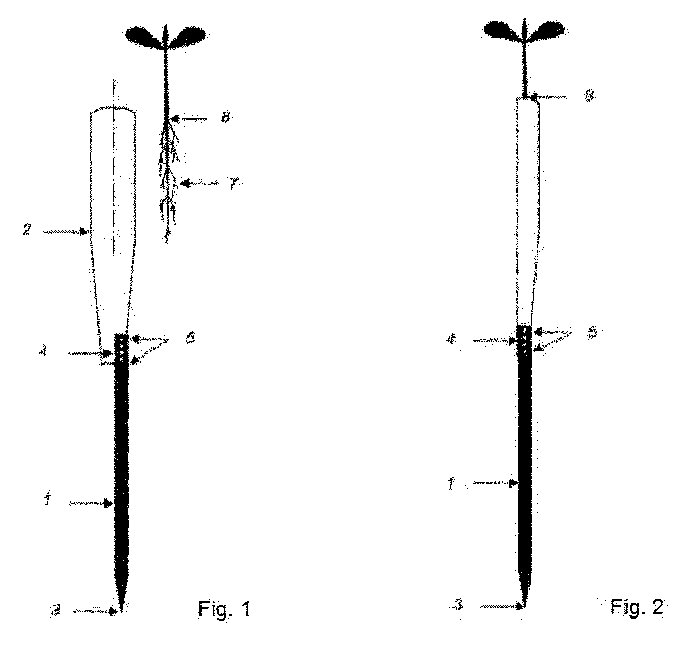

The pricking-out device for the method according to the present invention is described in the following examples, and schematically depicted in Figures. Figure 1 shows a side view of the open pricking-out device before the insertion of the seedling's root system. Figure 2 shows a side view of the closed pricking-out device with the seedling placed inside.

### Examples of embodiments of carrying out the Invention

The present invention is further described in the following example which should not be construed as limiting.

The pricking-out device for the method according to the present invention comprises a needle-shaped prick 1 with a point 3 on its first end (in the depicted position, it is the bottom end). The prick 1 is a longitudinal body, sufficiently resistant against bending, as will be explained further in the text. The second end (in the depicted position, it is the upper end) of the prick 1 is provided with a longitudinal guiding means 2 for the plant root system 7 of the pricked-out plant 8. The prick 1 has a suitable size and form of its cross-section, which can be for example circular, oval, triangular, hexagonal or combined etc.

The guiding means 2 are fixed to the prick 1 in a suitable way. In Figures 1 and 2, the prick 1 is provided with at least one transverse pass-through opening 5 and the guiding means 2 are attached to the prick 1 by for example sewing through the opening 4.

Alternatively, the fixation can be achieved by means of a rivet, a screw joint, glue, a fuse joint or by another suitable demountable or non-demountable attachment means.

In the depicted example, the prick 1 is flattened in the section of attachment to the guiding means 2. In a non-depicted example, the prick 1 in the section of attachment to the guiding means 2 is suitably shaped in another way or it has the original cross-section of the body of the prick 1.

The guiding means 2 contain a substantially flexible longitudinal flat body, which enables to enwrap, surround or enclose the root system 7 of the pricked out plant 8 prior to its introduction into the potting soil. In the depicted example, the guiding means 2 consist of a band of a suitable material, for example of a plastic material, which is longitudinally bent into two parallel areas, forming a shape of letter "V" in their cross-section. One end (in the depicted figure, it is the bottom part) of the guiding means 2 is attached to the second end (upper part in the figure) of the prick 1 by enwrapping it from the two opposite sides. In a non-depicted example, the guiding means 2 can comprise three or any other suitable number of parallel longitudinally arranged bands. The guiding means 2 are composed of either one object, or of a set of parallel objects attached to the second end of the prick 1.

The above described attachment of the guiding means 2 to the second end of the prick 1 allows for enclosing of the elongated root system 7 of the pricked out plant 8 in a straightened position into the guiding means 2, whereas the guiding means 2 maintain the root system 7 of the pricked out plant 8 in the straightened position during the passage of the pricking-out device containing the pricked-out plant 8 through a layer of the potting soil. During the passage of the pricking-out device containing the enclosed root system 7 of the pricked-out plant 8 through the layer of the potting soil, the root system 7 of the pricked-out plant 8 is maintained inside the guiding means 2, by for example pressing of the opposite sides of the guiding means 2, between which the root system 7 of the pricked-out plant 8 is placed. The pricked-out plant 8 is therefore inserted into the potting soil with no risk of bending of the root system 7. Once the pricking-out device containing the pricked-out plant 8 reaches the desired depth in the potting soil, the root system 7 is released from the guiding means 2, for example by releasing the pressure on the opposite sides of the guiding means 2, and the root system 7 of the pricked-out plant 8 remains in the desired depth of the potting soil. The pricked-out plant 8 stays in the place in the potting soil where it was released, having its root system 7 straight, and the pricking-out device containing the guiding means 2 continues its movement in the same direction as during the introduction of the plant through the whole layer of the potting soil, for example through an open bottom of a flowerpot or a planter or a container etc. After its passage through the layer of potting soil, the pricking-out device is ready for inserting a root system 7 of another plant. The open bottom of a flowerpot, a planter or a container is to be understood as such a modification of the bottom which enables the passage of the pricking-out device through the layer of potting soil and through the bottom of the flowerpot, the planter or the container, etc., using a unidirectional movement of the pricking-out device from the top to the bottom of the flowerpot, planter or container. The open bottom can be, e.g., a hole in the bottom part of the flowerpot, planter or container. The prick 1 serves as a cavity maker in the potting soil, while the guiding means 2 serves to place, to hold and to guide in a controlled manner the root system 7 of the pricked-out plant 8 in the potting soil.

The present invention is not to be limited to the above described embodiments. It is evident that the basis of the invention is the provision and use of a pricking-out device which contains one part serving to form a cavity in the potting soil, and another part serving to place, to hold, to guide and to release in a controllable manner the root system 7 of the pricked-out plant 8 in the potting soil.

### Industrial Applicability

The present invention is applicable mainly in tree nurseries. It increases the quality of the production of enwrapped seedlings and it improves their root systems. Concerning this type of seedling material, it increases the efficiency of utilization of production surfaces. It also increases the efficiency of seedling production when seeds with reduced germinability are used. It also improves the quality of transplanting of plant cuttings.

## Claims

1. A method of pricking out plants including a step of inserting the root system of a pricked-out plant into growing substrate, **characterized in that** a pricking-out device comprising guiding means is provided, the root system (7) of the pricked-out plant (8) is inserted in a straightened position into guiding means (2) of a pricking-out device, whereupon the pricking-out device together with the root system (7) of the pricked-out plant (8) is inserted into a layer of growing substrate by a unidirectional movement and passed through the layer of the growing substrate by the unidirectional movement, whereas in a pre-determined depth in the layer of growing substrate the root system (7) is released from the guiding means (2) of the pricking-out device, and said pricking-out device then continues to move by the same unidirectional movement out of the growing substrate.

2. The method according to claim 1, wherein the pricking-out device continues to move by the unidirectional movement out of the growing substrate by passing through the whole layer of growing substrate as well as through a bottom of a flowerpot, a planter or a container.

3. The method according to claim 1 or 2, wherein the pricking out device moves by a unidirectional vertical movement from top to bottom of the flowerpot, planter or container.

4. The method according to any one of the preceeding claims, wherein the pricking-out device comprises a prick (1) having on one end the longitudinal guiding means (2) for guiding the root system (7) of the pricked out plant (8).

5. The method according to claim 4, wherein the guiding means (2) of the pricking-out device are attached to the prick (1) by a non-demountable attachment means.

6. The method according to claim 4, wherein the guiding means (2) of the pricking-out device are attached to the prick (1) by a demountable attachment means.

7. The method according to any one of the claims 4 to 6, wherein the prick (1) of the pricking-out device in the region of attachment to the guiding means (2) is shaped, preferably it is flattened.

8. The method according to any one of the claims 4 to 7, wherein the prick (1) of the pricking-out device in the region of attachment to the guiding means (2) contains at least one transverse pass-through opening (5).

9. The method according to any one of the claims 4 to 8, wherein the guiding means (2) of the pricking-out device are formed by a flexible longitudinal flat body.

10. The method according to claim 9, wherein the guiding means (2) are formed by a band of material, which is longitudinally bent into two parallel areas, forming a letter "V" in their cross-section.

## Patentansprüche

1. Methode des Pikierens von Pflanzen, umfassend den Schritt des Einlegens des Wurzelsystems der zu pikierenden Pflanze in das Anbausubstrat **dadurch gekennzeichnet, dass** eine Pikiervorrichtung gewährt wird, einschliessend ein Führungselement, wobei das Wurzelsystem (7) der zu pikierenden Pflanze (8) in aufgerichtetem Zustand in das Führungselement (2) der Pikiervorrichtung eingelegt wird, die Pikiervorrichtung mit dem Wurzelsystem (7) der zu pikierenden Pflanze (8) anschliessend in einer einseitigen Bewegung in die Anbausubstratschicht eingeführt und in einer einseitigen Bewegung durch die Anbausubstratschicht gezogen wird, wobei das Wurzelsystem (7) in einer bestimmten Anbausubstrattiefe vom Führungselement (2) der Pikiervorrichtung losgelassen wird und diese Pikiervorrichtung durch die Fortsetzung ihrer einseitigen Bewegung anschliessend aus dem Anbausubstrat herausgenommen wird.

2. Methode nach dem Anspruch 1, wobei die Pikiervorrichtung ihre einseitige Bewegung aus dem Anbausubstrat heraus dadurch fortsetzt, dass sie durch die ganze Anbausubstratschicht, sowie auch durch den Boden des Blumentopfes, des Aussaatbehälters oder des Containers hindurchgeht.

3. Methode nach dem Anspruch 1 oder 2, wobei sich die Pikiervorrichtung in einseitiger vertikaler Bewegung von oben nach unten durch den Blumentopf, Aussaatbehälter oder den Container bewegt.

4. Methode nach einem der vorherigen Ansprüche, wobei die Pikiervorrichtung einen Stecher (1) einschliesst, der an einem Ende mit einem länglichen Führungselement (2) zur Führung des Wurzelsystems (7) der zu pikierenden Pflanzen (8) versehen ist.

5. Methode nach dem Anspruch 4, wobei das Führungselement (2) der Pikiervorrichtung mit einem unlösbaren Anschlussglied am Stecher (1) befestigt ist.

6. Methode nach dem Anspruch 4, wobei das Führungselement (2) der Pikiervorrichtung mit einem lösbaren Anschlussglied am Stecher (1) befestigt ist.

7. Methode nach einem der Ansprüche 4 bis 6, wobei der Stecher (1) der Pikiervorrichtung im Anschlussbereich des Führungselements (2) geformt ist, vorzugsweise abgeflacht ist.

8. Methode nach einem der Ansprüche 4 bis 7, wobei der Stecher (1) der Pikiervorrichtung im Anschlussbereich des Führungselementes (2) mindestens ein durchgehendes Querloch (5) einschliesst.

9. Methode nach einem der Ansprüche 4 bis 8, wobei das Führungselement (2) der Pikiervorrichtung aus einem biegsamen länglichen Flachkörper besteht.

10. Methode nach dem Anspruch 9, wobei das Führungselement (2) aus einem Materialband besteht, das länglich in zwei parallele Flächen gebogen ist, welche im Querschnitt die "V"-Form aufweisen.

## Revendications

1. Procédé de repiquage des plantes comprenant la phase d'introduction du système racinaire du transplant dans le substrat de culture, **caractérisé en ce que** l'on dispose d'un dispositif pour le repiquage comprenant un guide, le système (7) racinaire du transplant (8) étant placé en position relevée dans le guide (2) du dispositif pour le repiquage, puis le dispositif pour le repiquage avec le système (7) racinaire du transplant (8) s'introduit d'un mouvement unidirectionnel dans la couche du substrat de culture et passe d'un mouvement unidirectionnel par la couche du substrat de culture où, à la profondeur prédéterminée du substrat de culture, le système (7) racinaire est libéré du guide (2) du dispositif pour le repiquage et tout en continuant dans son mouvement unidirectionnel ce dispositif pour le repiquage est ensuite retiré du substrat de culture.

2. Procédé selon la revendication 1, **caractérisé en ce que** le dispositif pour le repiquage continue dans son mouvement unidirectionnel vers l'extérieur du substrat de culture en passant par la couche entière du substrat de culture, ainsi qu'également par le fond du pot, du godet ou du container.

3. Procédé selon les revendications 1 ou 2, **caractérisé en ce que** le dispositif pour le repiquage passe de son mouvement unidirectionnel vertical du haut en bas par le pot, le godet ou le container.

4. Procédé selon l'une quelconque des revendications précédentes, **caractérisé en ce que** le dispositif de repiquage comprend un pointeau (1) doté à l'une de ses extrémités d'un guide (2) longitudinal pour le guidage du système (7) racinaire du transplant (8).

5. Procédé selon la revendication 4, **caractérisé en ce que** le guide (2) du dispositif pour le repiquage est fixé sur le pointeau (1) par un dispositif de fixation non-démontable.

6. Procédé selon la revendication 4, **caractérisé en ce que** le guide (2) du dispositif pour le repiquage est fixé sur le pointeau (1) par un dispositif de fixation démontable.

7. Procédé selon l'une quelconque des revendications 4 à 6, **caractérisé en ce que** le pointeau (1) du dispositif pour le repiquage est profilé au niveau de la fixation du guide (2), de préférence aplati.

8. Procédé selon l'une quelconque des revendications 4 à 7, **caractérisé en ce que** le pointeau (1) du dispositif pour le repiquage contient au moins un orifice (5) transversal de passage au niveau de la fixation du guide (2).

9. Procédé selon l'une quelconque des revendications 4 à 8, **caractérisé en ce que** le guide (2) du dispositif pour le repiquage est formé d'un corps longitudinal plat flexible.

10. Procédé selon la revendication 9, **caractérisé en ce que** le guide (2) est formé d'une bande de matériel, laquelle est repliée tout au long en deux surfaces parallèles formant en coupe transversale le profil de la lettre « V ».
